# EUROPEAN PATENT APPLICATION

(11) **EP 3 970 704 A1**
(43) Date of publication of application: **23.03.2022**
(21) Application number: 20805274.6
(22) Date of filing: 15.05.2020
(51) Int. Cl.: A61K 9/70, A61K 47/32, A61K 31/445, A61P 25/28

(54) **PREPARATION FOR PERCUTANEOUS ABSORPTION COMPRISING HIGH DOSE OF DONEPEZIL OR SALT THEREOF**

(30) Priority: 15.05.2019 KR 20190056741
(71) Applicant: Daewoong Pharmaceutical Co., Ltd., Hwaseong-si Gyenoggi-do 18623 (KR)
(72) Inventor: LEE, Seoyeo, Suwon-si Gyeonggi-do 16675 (KR); KIM, Gyoung Won, Yongin-si Gyeonggi-do 17009 (KR); KIM, Gwan Young, Seoul 07292 (KR)
(74) Representative: ABG Intellectual Property Law, S.L.
(86) International application number: PCT/KR2020/006437
(87) International publication number: WO 2020/231227

(57) **Abstract**

The present invention relates to a preparation for percutaneous absorption, comprising a drug-containing matrix layer having a single-layered structure, wherein a high dose of donepezil or a pharmaceutically acceptable salt thereof is comprised in an amount of 10 to 20 wt% with respect to the total weight of the drug-containing matrix layer, thereby reducing the size of a formulation. Since the preparation for percutaneous absorption according to the present invention has a single-layered structure, preparation thereof at a production site is easy and manufacturing costs can be reduced as compared to preparations for percutaneous absorption having a multi-layered structure. In addition, in spite of containing a high dose of donepezil, no crystallization of donepezil occurs even during long-term storage of the preparation, and the preparation continuously exhibits high skin permeability for a long period of time. Above all, due to a high drug dose per unit area, the size of a formulation is reduced as compared to that of conventional formulations, and thus patient medication compliance can be remarkably increased.

## Description

### [Technical Field]

The present invention relates to a preparation for percutaneous absorption, comprising a drug-containing matrix layer having a single-layered structure, wherein a high dose of donepezil or a pharmaceutically acceptable salt thereof is comprised in an amount of 10 to 20 wt% with respect to the total weight of the drug-containing matrix layer, thereby reducing the size of a formulation.

### [Background Art]

Patients with Alzheimer's dementia have significantly reduced ability to secrete cholinergic neurons and acetylcholine throughout the cerebral cortex. Therefore, it is known that the cognitive ability of patients with Alzheimer's dementia can be improved by increasing acetylcholine in the brain. Acetylcholinesterase inhibitors are used in the treatment of Alzheimer's dementia by suppressing acetylcholinesterase (AChE) to increase the amount of acetylcholine in the brain. As an acetylcholinesterase inhibitor developed to date, tacrine (1993), donepezil (1996), rivastigmine (1999), galantamine (2001) and the like were approved by the Food and Drug Administration (FDA) and sold. For example, a donepezil-containing oral tablet Aricept^{™} Tablet (Eisai Co., Ltd.), is a preparation to be taken once a day before bedtime, and tablets with doses of 5 mg, 10 mg, and 23 mg are commercially available.

However, it is known that upon oral administration, a donepezil-containing oral tablet causes gastrointestinal side effects such as diarrhea, nausea, loss of appetite, and muscle cramps in some patients. Further, since the drug needs be repeatedly taken once a day for patients with Alzheimer's disease before bedtime, the convenience of taking the drug deteriorates, so that it is difficult to exhibit a continuous pharmacological effect.

Therefore, studies have been conducted on various preparations for percutaneous absorption, containing donepezil or a salt thereof. For example, various prior documents such as U.S. Patent No. 6,815,454, U.S. Patent Publication Nos. 2009/0175929, 2010/0080842, and 2012/0207816, International Patent Publication No. WO 2003/032960, Korean Patent Publication Nos. 10-2009-0101667, 10-2011-0030349, and 10-2011-0109995 disclose preparations for percutaneous absorption, containing donepezil or a salt thereof.

Through Korean Patent No. 10-1485822, the present inventors also disclosed a preparation for percutaneous absorption, comprising a drug-containing matrix layer having a single-layered structure, wherein the drug-containing matrix layer comprises a mixture of high-molecular weight polyisobutylene and low-molecular weight polyisobutylene as an adhesive, and also comprises 3 wt% or less of a permeation enhancer.

However, although Korean Patent No. 10-1485822 discloses a preparation for percutaneous absorption, which is capable of continuous drug release for 7 days or more after one application even with the use of a small amount of permeation enhancer, the content of a drug in a drug-containing matrix in the preparation for percutaneous absorption of the patent is only 5 to 10 wt%. Since a preparation for percutaneous absorption may generate solid crystals during storage in a matrix, thereby causing a decrease in adhesive strength, non-uniformity of skin permeability, and storage problems, it is difficult to contain the drug at a high concentration, and the above patent also could not solve the technical problems.

### [Disclosure]

### [Technical Problem]

The present invention has been made in an effort to provide a preparation for percutaneous absorption, comprising a drug-containing matrix layer having a single-layered structure, which causes no crystallization of donepezil even during long-term storage in spite of containing a high dose of donepezil, continuously exhibits high skin permeability for a long period of time (at least 7 days or more), and can increase patient medication compliance because the size of a formulation is reduced as compared to that of conventional formulations due to a high drug dose per unit area.

### [Technical Solution]

To solve the problem, the present invention provides a preparation for percutaneous absorption, comprising a support layer, a drug-containing matrix layer and a release layer,
wherein the drug-containing matrix layer comprises:
   (a) 10 to 20 parts by weight of donepezil or a pharmaceutically salt thereof as an active ingredient with respect to a total of 100 parts by weight of the drug-containing matrix layer,
   (b) 30 to 55 parts by weight of a polyisobutylene mixture of high-molecular weight polyisobutylene having a viscosity average molecular weight of 400,000 to 3,000,000 and low-molecular weight polyisobutylene having a viscosity average molecular weight of 40,000 to 100,000 as an adhesive with respect to a total of 100 parts by weight of the drug-containing matrix layer, and
   (c) 0.5 to 2.5 parts by weight of a crystallization inhibitor with respect to a total of 100 parts by weight of the drug-containing matrix layer, and
wherein a dose per unit area of donepezil or the pharmaceutically acceptable salt thereof in the preparation for percutaneous absorption is 1.0 to 2.0 mg/cm².

The preparation for percutaneous adsorption according to the present invention is characterized by having a higher content of donepezil or a pharmaceutically acceptable salt thereof in the drug-containing matrix layer and having a higher dose per unit area of donepezil or a pharmaceutically acceptable salt thereof in the preparation for percutaneous absorption than the preparation for percutaneous absorption of donepezil reported in the related art.

The preparation for percutaneous absorption according to the present invention comprises a support layer, a drug-containing matrix layer and a release layer.

Among them, the drug-containing matrix layer essentially comprises: (a) donepezil or a pharmaceutically acceptable salt thereof as an active ingredient, (b) an adhesive and (c) a permeation enhancer.

Donepezil or a pharmaceutically acceptable salt thereof, which is an active ingredient of the preparation for percutaneous absorption according to the present invention, is comprised in an amount of 9 to 14 parts by weight with respect to a total of 100 parts by weight of the drug-containing matrix layer. Preparations for percutaneous absorption previously reported in the related art could not prevent formation of crystals in the matrix when the drug was contained in a high concentraion, but according to the present invention, it was revealed that even though 9 to 14 parts by weight of donepezil or a pharmaceutically acceptable salt thereof is contained with respect to a total of 100 parts by weight of the drug-containing matrix layer, stability can be maintained because crystals are not generated even though 6 months have passed under warm/room temperature conditions. In exemplary embodiments of the present invention, donepezil or a pharmaceutically acceptable salt thereof may be comprised in an amount of, but not limited to, any range within the numerical range of 9 to 14 parts by weight, for example, 9 to 13 parts by weight, 9 to 12 parts by weight, 9 to 11 parts by weight, 10 to 13 parts by weight, 10 to 12 parts by weight, and 10 to 11 parts by weight, with respect to a total of 100 parts by weight of the drug-containing matrix layer.

The present inventors have focused on the advantage that the size of a formulation can be reduced when the preparation for percutaneous absorption contains a drug at a high concentration. In the case of preparations for percutaneous absorption used instead of oral preparations, the size of a formulation may be a very important factor in the treatment of patients, particularly because the size of the formulation is also associated with the patient medication convenience. Therefore, in order to prepare a high dose of a preparation for percutaneous absorption of donepezil, which can reduce the size of the formulation, the present inventors set a high dose per unit area of the drug in the preparation for percutaneous absorption.

The preparation for percutaneous absorption according to the present invention may have a dose per unit area of donepezil or a pharmaceutically acceptable salt thereof in the preparation for percutaneous absorption of 1.0 to 2.0 mg/cm². For example, a preparation for percutaneous absorption with a formulation size of 50 cm² can comprise 50 to 100 mg of donepezil or a pharmaceutically acceptable salt thereof. Since the preparation for percutaneous absorption of donepezil with a small area and a high dose has a small area of the formulation exposed to the skin of a patient, it is possible not only to increase patient medication convenience and medication compliance, but also to minimize the possibility of skin problems of a patient caused by an adhesive, and the like, and simultaneously, the preparation for percutaneous absorption of donepezil contains a high dose of donepezil, and thus can continuously release the drug even for a long period of time of 7 days or more, thereby providing patient medication convenience, which is differentiated from an oral preparation taken once a day. In one exemplary embodiment of the present invention, a dose per unit area of donepezil or a pharmaceutically acceptable salt thereof in the preparation for percutaneous absorption is not limited to, but may be any range within the numerical range of 1.0 to 2.0 mg/cm², for example, 1.0 to 1.9 mg/cm², 1.0 to 1.8 mg/cm², 1.0 to 1.6 mg/cm², 1.0 to 1.5 mg/cm², 1.0 to 1.4 mg/cm², 1.0 to 1.3 mg/cm², 1.0 to 1.2 mg/cm², 1.1 to 1.5 mg/cm², 1.1 to 1.4 mg/cm², and 1.1 to 1.3 mg/cm².

In the preparation for percutaneous absorption according to the present invention, the drug dose per application can be adjusted by adjusting the size of the formulation. For example, the preparation for percutaneous absorption according to the present invention may be a preparation having a size of 30 cm² to 150 cm². In one exemplary embodiment of the present invention, the preparation for percutaneous absorption according to the present invention may be a preparation having a size of 40 cm² to 120 cm². For example, a preparation for percutaneous absorption with a size of 40 cm² to 60 cm² may comprise 40 to 120 mg of donepezil or a pharmaceutically acceptable salt thereof. Alternatively, a preparation for percutaneous absorption with a size of 80 cm² to 120 cm² may comprise 80 to 240 mg of donepezil or a pharmaceutically acceptable salt thereof. The preparation for percutaneous absorption of donepezil with the corresponding dose may be administered once a week in place of an oral preparation administered once a day, such as an Aricept^{™} tablet.

In one exemplary embodiment of the present invention, it is possible to exhibit bioequivalent levels of an area under the blood concentration-time curve (AUC) and a maximum blood concentration (Cₘₐₓ) when an Aricept^{™} tablet having an active ingredient dose of 8 to 10 parts by weight based on 100 parts by weight of the dose of donepezil in the preparation for percutaneous absorption of donepezil or a pharmaceutically acceptable salt thereof is administered once a week as compared to the case when the Aricept^{™} tablet is administered once a day for 7 days.

Meanwhile, in the preparation for percutaneous absorption according to the present invention, the drug-containing matrix layer has a single-layered structure, and the single-layered structure is achieved using two types of polyisobutylene as an adhesive, that is, a mixture of high-molecular weight polyisobutylene having a viscosity average molecular weight of 400,000 to 3,000,000 and low-molecular weight polyisobutylene having a viscosity average molecular weight of 40,000 to 100,000 as an adhesive. When high-molecular weight polyisobutylene is used alone as an adhesive, the adhesive strength is so strong that mass production is difficult, and it may be difficult to remove a release liner from the obtained preparation for percutaneous absorption. The mixture of the high-molecular weight and low-molecular weight polyisobutylene adhesives forms a matrix of a drug-containing matrix layer.

That is, the drug-containing matrix layer is formed by uniformly dispersing donepezil or a pharmaceutically acceptable salt thereof in the mixture of the high-molecular weight and low-molecular weight polyisobutylene adhesives. Therefore, the preparation for percutaneous absorption according to the present invention can simplify the preparation process as compared to preparations for percutaneous absorption having a multi-layered structure, so that the scale-up is easy and economic costs required for the process can be reduced. In addition, the preparation for percutaneous absorption according to the present invention can maintain the skin permeability of a drug while stably adhering to the skin of a patient for a long period of time (7 days or more).

The high-molecular weight polyisobutylene has a viscosity average molecular weight of 400,000 to 3,000,000, and may have a viscosity average molecular weight of, for example, 800,000 to 2,500,000, 800,000 to 2,200,000, 800,000 to 2,000,000, 900,000 to 1,800,000, 900,000 to 1,500,000, 900,000 to 1,300,000, and 1,000,000 to 1,300,000. Commercially available high-molecular weight polyisobutylene, for example, Oppanol^{™} N 50, Oppanol^{™} N 80, Oppanol^{™} N 100, Oppanol^{™} N 150 (BASF, Germany) and the like may also be used, if necessary.

The low-molecular weight polyisobutylene has a viscosity average molecular weight of 40,000 to 100,000, and may have a viscosity average molecular weight of, for example, 40,000 to 90,000, 45,000 to 85,000, 45,000 to 75,000, 45,000 to 70,000, 45,000 to 65,000, 47,000 to 65,000, and 50,000 to 60,000. Commercially available low-molecular weight polyisobutylene, for example, Oppanol^{™}B 10, Oppanol^{™}B 11, Oppanol^{™}B 12, Oppanol^{™}B 13, Oppanol^{™}B 14, Oppanol^{™}B 15 (BASF, Germany) and the like may also be used, if necessary.

In one exemplary embodiment of the present invention, the high-molecular weight polyisobutylene may have a viscosity average molecular weight of 800,000 to 2,600,000. In another exemplary embodiment of the present invention, the low-molecular weight polyisobutylene may have a viscosity average molecular weight of 40,000 to 85,000.

In still another exemplary embodiment of the present invention, the adhesive may be a mixture of high-molecular weight polyisobutylene having a viscosity average molecular weight of 800,000 to 2,600,000 and low-molecular weight polyisobutylene having a viscosity average molecular weight of 40,000 to 85,000.

In yet another exemplary embodiment of the present invention, a weight ratio of the high-molecular weight polyisobutylene and the low-molecular weight polyisobutylene may be 1:0.5 to 1:2, for example, about 1:1, but is not limited thereto. The adhesive may be present in a range of, but not limited to, 35 to 50 parts by weight, for example, 40 to 48 parts by weight, with respect to a total of 100 parts by weight of the drug-containing matrix layer.

In the preparation for percutaneous absorption according to the present invention, the crystallization inhibitor is present in an amount of 0.5 to 2.5 parts by weight, and present in a range of preferably 0.5 to 1.5 parts by weight, with respect to a total of 100 parts by weight of the drug-containing matrix layer. The crystallization inhibitor comprises, but is not limited to, povidone, copovidone, and the like. As a crystallization inhibitor capable of achieving long-term formulation stability while including a dose per unit area of donepezil or a pharmaceutically acceptable salt thereof in the preparation for percutaneous absorption of 1.0 to 2.0 mg/cm², preferably, povidone having a molecular weight of about 40,000 to 3,000,000, for example, Povidone K30, Povidone K60, Povidone K90, Povidone K120, or copovidone may be used. The crystallization inhibitor may be included in an amount of 1.0 to 1.5 parts by weight with respect to a total of 100 parts by weight of the drug-containing matrix layer.

In one exemplary embodiment of the present invention, the crystallization inhibitor may be povidone. For example, 1.0 to 1.5 parts by weight of povidone may be comprised as a permeation enhancer with respect to a total of 100 parts by weight of the drug-containing matrix.

The drug-containing matrix layer may further comprise one or more additives selected from the group consisting of a stabilizer, a tackifier, and a softening agent, which are typically used in the field of a preparation for percutaneous absorption.

The stabilizer serves to enhance the drug stability of donepezil used in preparations for percutaneous absorption, and for example, ascorbyl palmitate may be used, but the stabilizer is not limited thereto. The amount of stabilizer used is not particularly limited, and may be 0.001 to 2 parts by weight, for example, 0.005 to 1 part by weight, 0.01 to 0.5 part by weight, 0.01 to 0.1 part by weight with respect to a total of 100 parts by weight of the drug-containing matrix.

Furthermore, a thickener comprises a typical viscosity enhancer, and includes, for example, a hydrocarbon resin (for example, commercially available Pinecrystal KE-311, Kristalex^{™} F85, Piccota ^{™} 1020, 1095, Escorez^{™} 5000, Koresin^{™} and the like). The amount of thickener used varies depending on the type of thickener used, but may be 15 to 35 parts by weight, preferably about 20 to 30 parts by weight with respect to a total of 100 parts by weight of the drug-containing matrix layer.

As the softening agent, a mineral oil, for example, paraffin liquid, and the like may be used, and the amount of softening agent used may be 10 to 20 parts by weight, for example, 15 to 20 parts by weight, with respect to a total of 100 parts by weight of the drug-containing matrix layer.

The preparation for percutaneous absorption according to the present invention may be prepared by forming the aforementioned drug-containing matrix layer on a release layer and then forming a support layer thereon. As the release layer, a release liner or a laminate thereof, which is typically used in the field of a preparation for percutaneous absorption, may be used, and for example, a film, a paper, or a laminate thereof such as polyethylene, polyester, polyvinyl chloride, and polyvinylidene chloride to which a silicone resin or a fluorine resin is applied may be used. Further, a drug non-absorbable and flexible material typically used in the field of a preparation for percutaneous absorption may be used as the support layer (or also referred to as a backing membrane), and for example, a polyolefin, polyether, a multi-layer ethylene vinyl acetate film, polyester, polyurethane and the like may be used. Further, by forming a non-woven fabric layer (overlay-tape or overlay-film) on the support layer, an adhesion force for long-term attachment can be maintained. In addition, as the non-woven fabric layer, a flexible material typically used in the field of a preparation for percutaneous absorption may be used, and for example, polyurethane, polyester, polyolefin, and the like may be used. For example, the preparation for percutaneous absorption according to the present invention may be prepared by homogeneously mixing donepezil or a pharmaceutically acceptable salt thereof, high-molecular weight polyisobutylene, low-molecular weight polyisobutylene, a stabilizer, an oil, and a thickener using a roller mixer and the like, and then applying the resulting mixture on a release liner coated with silicone, and then laminating a support layer.

### [Advantageous Effects]

Since the preparation for percutaneous absorption according to the present invention has a single-layered structure, preparation thereof at a production site is easy and manufacturing costs can be reduced as compared to preparations for percutaneous absorption having a multi-layered structure. In addition, in spite of containing a high dose of donepezil, no crystallization of donepezil occurs even during long-term storage of the preparation, and the preparation continuously exhibits high skin permeability for a long period of time (at least 7 days or more) in spite of including a small amount of a permeation enhancer. Above all, due to a high drug dose per unit area, the size of a formulation is reduced as compared to that of conventional formulations, and thus patient medication compliance can be remarkably increased.

### [Description of Drawings]

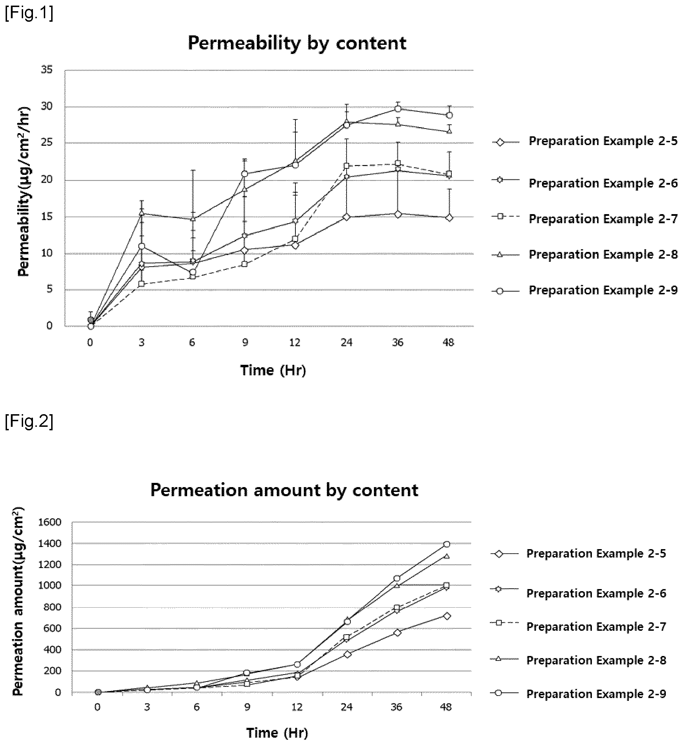

FIGS. 1 and 2 illustrate the results of measuring the skin permeability and permeation amount by applying the preparations for percutaneous absorption of Preparation Examples 2-5 to 2-9 to the skin of hairless mice.

### [Modes of the Invention]

Hereinafter, the present invention will be described in detail through Examples and Experimental Examples. However, these Examples and Experimental Examples are provided only for exemplifying the present invention, and the present invention is not limited to these Examples and Experimental Examples.

### [Examples]

### [Preparation Examples 1 to 4]

Preparations for percutaneous absorption were prepared according to the ingredients and contents in the following Table 1. Hereinafter, the numerical values in the table associated with the Production Examples indicate the weight (mg) of the raw material contained per preparation for percutaneous absorption having a size of about 47.6 cm². Specifically, a donepezil free base, polyisobutylene having a viscosity average molecular weight of about 1,110,000 [g/mol] (Oppanol^{™} N100, BASF, Germany) and polyisobutylene having a viscosity average molecular weight of about 55,000 [g/mol] (Oppanol^{™}B12, BASF, Germany), polyvinylpyrrolidone (Povidone K-30, K-25, K-90) or copovidone, isopropanol, toluene, a mineral oil, and a hydrocarbon resin (Pinecrystal KE-311,) were homogeneously mixed using a roller mixer. After air bubbles were removed from the obtained mixture, the mixture was applied to a release liner coated with silicone and dried. A preparation for percutaneous absorption containing donepezil was prepared by laminating a polyethylene film to form a backing membrane.

**[Table 1]**

| **Name of raw material** | **Preparation Example 1** | **Preparation Example 2** | **Preparation Example 3** | **Preparation Example 4** |
|---|---|---|---|---|
| Donepezil | 54 | 54 | 54 | 54 |
| Ascorbyl palmitate | 0.1 | 0.1 | 0.1 | 0.1 |
| Toluene (preparation solvent) | 200.0 | 200.0 | 200.0 | 200.0 |
| Hydrocarbon resin | 134.0 | 134.0 | 134.0 | 134.0 |
| Toluene (tackifier solvent) | 89.0 | 89.0 | 89.0 | 89.0 |
| Povidone K25 (PVP K25) | 5.6 | - | - | - |
| Povidone K30 (PVP K30) | - | 5.6 | - | - |
| Povidone K90 (PVP K90) | - | - | 5.6 | - |
| Copovidone | - | - | - | 5.6 |
| Isopropanol | 39.2 | 39.2 | 39.2 | 39.2 |
| Paraffin liquid (Mineral oil) | 86.8 | 86.8 | 86.8 | 86.8 |
| Polyisobutylene pressure adhesive (hereinafter, Duro-Tak 87-6919) | 968.3 | 968.3 | 968.3 | 968.3 |
| Sub-Total (Wet basis) | 1577 | 1577 | 1577 | 1577 |
| Sub-Total (Dried basis) | 503.2 | 503.2 | 503.2 | 503.2 |

### Experimental Example 1: Evaluation of stability in Preparation Examples 1 to 4

After room temperature, acceleration, open acceleration, and freeze-thaw tests (storage at -20°C/room temperature for 24 hours repeated three times) were performed on the prepared preparations for percutaneous absorption, preparation stability was evaluated by allowing the preparations to stand under open acceleration conditions for 4 weeks.

**[Table 2]**

| Preparation Example | Stability | | | |
|---|---|---|---|---|
| | Room temperature | Acceleration | Open acceleration | Freeze thaw open acceleration |
| Preparation Example 1 | X | O (week 2) | O (week 4) | O (week 4) |
| Preparation Example 2 | X | O (week 4) | X | O (week 4) |
| Preparation Example 3 | X | X | O (week 4) | O (week 4) |
| Preparation Example 4 | X | X | X | X |

As can be seen in Table 2, in the case of Preparation Example 1, crystals were formed in the preparation for percutaneous absorption at week 2 under the accelerated conditions, so it was evaluated that the compositions of Preparation Examples 2 to 4 were more preferable than Preparation Example 1. In particular, in the case of Preparation Example 2, almost no crystal generation was observed, so an additional experiment was performed based on the composition of Preparation Example 2.

### [Preparation Examples 2-1 to 2-4]

An attempt was made to test how preparation stability changes by adjusting the content of crystallization inhibitor in the composition of Preparation Example 2. Thus, Preparation Examples 2-1 to 2-4 were prepared by equally maintaining the content of the drug and the content of the other excipients and differently adjusting only the content of the crystallization inhibitor in the composition of Preparation Example 2.

**[Table 3]**

| **Name of raw material** | **Preparation Example 2-1** | **Preparation Example 2-2** | **Preparation Example 2-3** | **Preparation Example 2-4** |
|---|---|---|---|---|
| Donepezil | 54 | 54 | 54 | 54 |
| Ascorbyl palmitate | 0.1 | 0.1 | 0.1 | 0.1 |
| Toluene (preparation solvent) | 200.0 | 200.0 | 200.0 | 200.0 |
| Hydrocarbon resin | 134.0 | 134.0 | 134.0 | 134.0 |
| Toluene (tackifier solvent) | 89.0 | 89.0 | 89.0 | 89.0 |
| Povidone K30 (PVP K30) | 2.5 | 4.0 | 5.6 | 7.6 |
| Isopropanol | 39.2 | 39.2 | 39.2 | 39.2 |
| Paraffin liquid (Mineral oil) | 86.8 | 86.8 | 86.8 | 86.8 |
| Polyisobutylene pressure adhesive (hereinafter, Duro-Tak 87-6919) | 968.3 | 968.3 | 968.3 | 968.3 |
| Sub-Total (Wet basis) | 1573.9 | 1575.4 | 1577 | 1579 |
| Sub-Total (Dried basis) | 500.1 | 501.6 | 503.2 | 505.2 |

### Experimental Example 2: Evaluation of stability of Preparation Examples 2-1 to 2-4

The stability of Preparation Examples 2-1 to 2-4 was evaluated under the same conditions as in Experimental Example 1 for 4 weeks, and the results are shown in the following Table 4.

**[Table 4]**

| | Stability | | | |
|---|---|---|---|---|
| Preparation of patch | Room temperature | Acceleration | Open acceleration | Freeze thaw open acceleration |
| Preparation Example 2-1 | X | O (week 4) | O (week 2) | O (week 2) |
| Preparation Example 2-2 | X | X | X | O (week 4) |
| Preparation Example 2-3 | X | O (week 4) | X | O (week 4) |
| Preparation Example 2-4 | X | X | O (week 2) | O (week 4) |

As can be seen in Table 4, all of Preparation Examples 2-1 to 2-4 show generally good preparation stability.

### [Preparation Examples 2-5 to 2-9]

An attempt was made to test how preparation stability changed when the amount of drug loaded was changed in the composition of Preparation Example 2. Thus, Preparation Examples 2-5 to 2-9 were prepared by equally maintaining the content of the excipients and differently adjusting only the content of the drug as shown in the following Table 5 in the composition of Preparation Example 2.

**[Table 5]**

| **Name of raw material** | **Preparatio n Example 2-5** | **Preparatio n Example 2-6** | **Preparatio n Example 2-7** | **Preparatio n Example 2-8** | **Preparation Example 2-9** |
|---|---|---|---|---|---|
| Donepezil | 45.30 | 54.0 | 61.0 | 71.0 | 81.0 |
| Ascorbyl palmitate | 0.1 | 0.1 | 0.1 | 0.1 | 0.1 |
| Toluene (preparation solvent) | 200.0 | 200.0 | 200.0 | 200.0 | 200.0 |
| Hydrocarbon resin | 134.0 | 134.0 | 134.0 | 134.0 | 134.0 |
| Toluene (tackifier solvent) | 89.0 | 89.0 | 89.0 | 89.0 | 89.0 |
| Povidone K30 (PVP K30) | 5.6 | 5.6 | 5.6 | 5.6 | 5.6 |
| Isopropanol | 39.2 | 39.2 | 39.2 | 39.2 | 39.2 |
| Paraffin liquid (Mineral oil) | 86.8 | 86.8 | 86.8 | 86.8 | 86.8 |
| Polyisobutylene adhesive (hereinafter, Duro-Tak 87-6919) | 968.3 | 968.3 | 968.3 | 968.3 | 968.3 |
| Sub-Total (Wet basis) | 1568.3 | 1577 | 1584 | 1594 | 1604 |
| Sub-Total (Dried basis) | 494.5 | 503.2 | 510.2 | 520.2 | 530.2 |

### Experimental Example 3: Evaluation of stability of Preparation Examples 2-5 to 2-9

The stability of Preparation Examples 2-5 to 2-9 was evaluated under the same conditions as in Experimental Example 1 for 4 weeks, and the results are shown in the following Table 6.

**[Table 6]**

| Preparation of patch | Stability | | | |
|---|---|---|---|---|
| | Room temperature | Acceleration | Open acceleration | Freeze thaw open acceleration |
| Preparation Example 2-5 | X | X | X | X |
| Preparation Example 2-6 | X | O (week 4) | X | O (week 4) |
| Preparation Example 2-7 | X | O (week 4) | X | O (week 4) |
| Preparation Example 2-8 | X | X | O (week 4) | X |
| Preparation Example 2-9 | X | X | O (week 4) | O (week 4) |

In Preparation Examples 2-8 and 2-9, crystal generation was conspicuous, indicating poor preparation stability. Other Preparation Examples 2-5 to 2-7 showed relatively good stability.

### [Preparation Examples 2-10 to 2-13]

During a preliminary experiment, it was revealed that the change in co-solvent of the crystallization inhibitor at the time of preparation of the preparation for percutaneous absorption affects preparation stability. Thus, an attempt was made to test how preparation stability changed when the type of co-solvent was varied in the composition of Preparation Example 2. Thus, Preparation Examples 2-10 to 2-13 were prepared by equally maintaining the contents of the drug and the excipients and using only different types of co-solvent during the preparation of the crystallization inhibitor solution as shown in the following Table 7 in the composition of Preparation Example 2.

**[Table 7]**

| **Name of raw material** | **Preparation Example 2-10** | **Preparation Example 2-11** | **Preparation Example 2-12** | **Preparation Example 2-13** |
|---|---|---|---|---|
| Donepezil | 54 | 54 | 54 | 54 |
| Ascorbyl palmitate | 0.1 | 0.1 | 0.1 | 0.1 |
| Toluene (preparation solvent) | 200.0 | 200.0 | 200.0 | 200.0 |
| Hydrocarbon resin | 134.0 | 134.0 | 134.0 | 134.0 |
| Toluene (tackifier solvent) | 89.0 | 89.0 | 89.0 | 89.0 |
| Povidone K30 (PVP K30) | 5.6 | 5.6 | 5.6 | 5.6 |
| Ethanol | 39.2 | - | - | - |
| Butanol | - | 39.2 | - | - |
| Isopropanol | - | - | 39.2 | - |
| Propanol | - | - | - | 39.2 |
| Paraffin liquid (Mineral oil) | 86.8 | 86.8 | 86.8 | 86.8 |
| Polyisobutylene adhesive (Hereinafter, Duro-Tak 87- | 968.3 | 968.3 | 968.3 | 968.3 |
| 6919) | | | | |
| Sub-Total (Wet basis) | 1577 | 1577 | 1577 | 1577 |
| Sub-Total (Dried basis) | 503.2 | 503.2 | 503.2 | 503.2 |

### Experimental Example 4: Evaluation of stability of Preparation Examples 2-10 to 2-13

The stability of Preparation Examples 2-10 to 2-13 was evaluated under the same conditions as in Experimental Example 1 for 4 weeks, and the results are shown in the following Table 8.

**[Table 8]**

| Preparation of patch | Stability | | | |
|---|---|---|---|---|
| | Room temperature | Acceleration | Open acceleration | Freeze thaw open acceleration |
| Preparation Example 2-10 | X | O (week 2) | O (week 2) | O (week 2) |
| Preparation Example 2-11 | X | O (week 4) | O (week 2) | O (week 2) |
| Preparation Example 2-12 | X | O (week 4) | X | O (week 4) |
| Preparation Example 2-13 | X | O (week 4) | O (week 4) | O (week 4) |

As a result of the experiment, the crystal generation of Preparation Example 2-12 using isopropanol as a co-solvent was the least compared to other solvents.

### [Preparation Examples 2-14 to 2-16]

In order to confirm whether the content of co-solvent affected preparation stability when the preparation for percutaneous absorption was prepared, an attempt was made to test how preparation stability changed when the content of co-solvent in the composition of Preparation Example 2-12 was varied. Thus, Preparation Examples 2-14 to 2-16 were prepared by equally maintaining the contents of the drug and the excipients and using only different contents of co-solvent during the preparation of the crystallization inhibitor solution as shown in the following Table 9 in the composition of Preparation Example 2-12.

**[Table 9]**

| **Name of raw material** | **Preparation Example 2-14** | **Preparation Example 2-15** | **Preparation Example 2-16** |
|---|---|---|---|
| Donepezil | 54 | 54 | 54 |
| Ascorbyl palmitate | 0.1 | 0.1 | 0.1 |
| Toluene (preparation solvent) | 200.0 | 200.0 | 200.0 |
| Hydrocarbon resin | 134.0 | 134.0 | 134.0 |
| Toluene (tackifier solvent) | 89.0 | 89.0 | 89.0 |
| Povidone K30 (PVP K30) | 5.6 | 5.6 | 5.6 |
| Isopropanol | 16.8 | 39.2 | 84 |
| Paraffin liquid (Mineral oil) | 86.8 | 86.8 | 86.8 |
| Polyisobutylene pressure adhesive (hereinafter, Duro-Tak 87-6919) | 968.3 | 968.3 | 968.3 |
| Sub-Total (Wet basis) | 1554.6 | 1577 | 1621.8 |
| Sub-Total (Dried basis) | 503.2 | 503.2 | 503.2 |

### Experimental Example 5: Evaluation of stability of Preparation Examples 2-14 to 2-16

The stability of Preparation Examples 2-14 to 2-16 was evaluated under the same conditions as in Experimental Example 1 for 4 weeks, and the results are shown in the following Table 10.

**[Table 10]**

| Preparation of patch | Stability | | | |
|---|---|---|---|---|
| | Room temperature | Acceleration | Open acceleration | Freeze thaw open acceleration |
| Preparation Example 2-14 | X | O (week 4) | O (week 4) | O (week 2) |
| Preparation Example 2-15 | X | O (week 4) | X | O (week 4) |
| Preparation Example 2-16 | O (precipitation of crystals immediately after preparation of patch) | | | |

As a result of the experiment, in Preparation Example 2-16, crystals were precipitated immediately after the preparation of a patch so a stable preparation could not be obtained. It was observed that the stability of the preparation stability was secured in Preparation Examples 2-14 and 2-15.

### Experimental Example 6: Percutaneous dissolution test

The skin permeability and permeation amount were measured by applying the preparations for percutaneous absorption of Preparation Examples 2-5 to 2-9 to the skin of hairless mice.

Specifically, after each preparation for percutaneous absorption was adhered to the skin of hairless mice (6 to 8 weeks old) by removing the skin immediately before the test, the skin was fixed with a clamp in a flow-through diffusion cell, an isotonic phosphate buffer solution (pH 7.4) was put into a receptor and maintained at 32.5°C, and a sample was collected at 0, 3, 6, 9, 12, 24, 36, and 48 hours while stirring the solution at 600 rpm with a magnetic stirrer. The obtained sample was quantified using high performance liquid chromatography and an ultraviolet absorptiometer under the following analytical conditions.

### <Analytical conditions>

Column: Capcellpak MG C18, 4.6 X 150 mm, 5 µm
Mobile phase: after 2.12 g of sodium decanesulfonate was completely dissolved in 550 mL of water, 1 mL of 70% perchloric acid and 450 mL of an acetonitrile solution were added thereto. After the obtained solution was filtered, air bubbles were removed for 10 minutes using an ultrasonic cleaner and the resulting product was used as a mobile phase.
Column temperature: 35°C
Amount of sample injected: 5 µl
Ultraviolet absorptiometer: 271 nm
Flow rate: 1.0 mL/min

As a result of a percutaneous dissolution test of the preparations for percutaneous absorption of Preparation Examples 2-5 to 2-9, the permeability (µg/cm²/hr) is shown in the following Table 11 and FIG 1.

**[Table 11]**

| **Preparation Example** | **Time (hr)** | **0** | **3** | **6** | **9** | **12** | **24** | **36** | **48** |
|---|---|---|---|---|---|---|---|---|---|
| Preparation Example 2-5 | average | 0 | 8.1 | 8.6 | 10.5 | 11.2 | 14.9 | 15.4 | 14.8 |
| | STDEV | 0 | 7.9 | 7.0 | 7.8 | 7.1 | 6.8 | 5.8 | 3.9 |
| Preparation Example 2-6 | average | 0 | 8.6 | 8.8 | 12.4 | 14.3 | 20.5 | 21.3 | 20.6 |
| | STDEV | 0 | 5.6 | 4.3 | 5.3 | 5.3 | 5.2 | 3.8 | 3.3 |
| Preparation Example 2-7 | average | 0 | 5.8 | 6.7 | 8.5 | 11.9 | 21.9 | 22.2 | 20.8 |
| | STDEV | 0 | 1.9 | 5.4 | 5.8 | 5.9 | 3.7 | 3.1 | 3.1 |
| Preparation Example 2-8 | average | 0 | 15.5 | 14.7 | 18.7 | 22.6 | 28.0 | 27.7 | 26.6 |
| | STDEV | 0 | 1.7 | 6.7 | 3.9 | 5.7 | 1.3 | 0.8 | 0.9 |
| Preparation Example 2-9 | average | 0 | 10.9 | 7.2 | 20.9 | 22.1 | 27.5 | 29.7 | 28.9 |
| | STDEV | 0 | 1.4 | 3.1 | 1.9 | 4.4 | 2.8 | 1.0 | 1.2 |

Further, the permeation amount (µg/cm²) by content of donepezil is shown in Table 12 and FIG. 2.

**[Table 12]**

| **Sample (content)** | **Time (hr)** | **0** | **3** | **6** | **9** | **12** | **24** | **36** | **48** |
|---|---|---|---|---|---|---|---|---|---|
| Preparatio n Example 2-5 | average | 0 | 24.2 | 51.5 | 94.8 | 134.8 | 357.8 | 555.9 | 711.7 |
| | STDEV | 0 | 23.8 | 41.9 | 70.4 | 85.8 | 164.2 | 209.1 | 187.4 |
| Preparatio n Example 2-6 | average | 0 | 25.8 | 52.6 | 111.3 | 172.0 | 490.8 | 768.4 | 988.3 |
| | STDEV | 0 | 16.8 | 25.7 | 48.1 | 63.3 | 124.8 | 138.3 | 158.2 |
| Preparatio n Example 2-7 | average | 0 | 17.3 | 40.3 | 76.4 | 143.4 | 526.5 | 798.9 | 997.2 |
| | STDEV | 0 | 5.7 | 32.4 | 52.5 | 71.0 | 87.9 | 109.8 | 147.2 |
| Preparatio n Example 2-8 | average | 0 | 46.4 | 88.2 | 168.3 | 270.8 | 672.8 | 996.2 | 1277.4 |
| | STDEV | 0 | 5.1 | 40.0 | 35.0 | 68.7 | 30.9 | 29.0 | 43.2 |
| Preparatio n Example 2-9 | average | 0 | 32.7 | 43.3 | 188.5 | 264.8 | 659.3 | 1069.8 | 1389.4 |
| | STDEV | 0 | 4.2 | 18.6 | 17.0 | 53.3 | 68.4 | 34.8 | 56.6 |

### Experimental Example 7: Observation of related substances formation

In order to confirm the safety of the preparation for percutaneous absorption of Preparation Example 2-6, the contents of donepezil and a related substances thereof were quantified at a time point when 4 months after the production of the preparation had elapsed. As a result, the contents of donepezil and the related substances in the preparation for percutaneous absorption of Preparation Example 2-6 containing 54 mg of donepezil were measured as follows, and were confirmed to be included at reference amount levels.

**[Table 13]**

| **Item** | **Reference** | **Initial** | **4 months passed at room temperature** |
|---|---|---|---|
| Donepezil | 95 to 105% | 103.4% | 101.5% |
| BDNPZ^{∗} | NMT 0.2% | N.D | N.D |
| DDIMP^{∗∗} | NMT 0.2% | N.D | N.D |
| N-oxide | NMT 0.5% | 0.08% | 0.06% |
| Individual related substances | NMT0.2% | 0.04% | 0.06% |
| Total related substances | NMT 1.2% | 0.11% | 0.13% |

| | | | |
|---|---|---|---|
| ^{∗}BDNPZ : N-dibenzyl-4-[(dimethoxyindanon)-2-methyl]piperidine chloride ^{∗∗} DDIMP : 4-[(dimethoxyindanone)-2-methyl]piperidine | | | |

## Claims

1. A preparation for percutaneous absorption, comprising: a support layer, a drug-containing matrix layer and a release layer,
wherein the drug-containing matrix layer comprises:
(a) 9 to 14 parts by weight of donepezil or a pharmaceutically salt thereof as an active ingredient with respect to a total of 100 parts by weight of the drug-containing matrix layer,
(b) 30 to 55 parts by weight of a polyisobutylene mixture of high-molecular weight polyisobutylene having a viscosity average molecular weight of 400,000 to 3,000,000 and low-molecular weight polyisobutylene having a viscosity average molecular weight of 40,000 to 100,000 as an adhesive with respect to a total of 100 parts by weight of the drug-containing matrix layer, and
(c) 0.5 to 2.5 parts by weight of a crystallization inhibitor with respect to a total of 100 parts by weight of the drug-containing matrix layer, and
wherein a dose per unit area of donepezil or the pharmaceutically acceptable salt thereof in the preparation for percutaneous absorption is 1.0 to 2.0 mg/cm².

2. The preparation of claim 1, wherein donepezil or a pharmaceutically acceptable salt thereof is comprised in an amount of 10 to 13 parts by weight with respect to a total of 100 parts by weight of the drug-containing matrix layer.

3. The preparation of claim 1, wherein a dose per unit area of donepezil or the pharmaceutically acceptable salt thereof in the preparation for percutaneous absorption is 1.0 to 1.5 mg/cm².

4. The preparation of claim 1, wherein the high-molecular weight polyisobutylene has a viscosity average molecular weight of 800,000 to 2,600,000.

5. The preparation of claim 1, wherein the low-molecular weight polyisobutylene has a viscosity average molecular weight of 40,000 to 85,000.

6. The preparation of claim 1, wherein the adhesive is a mixture of high-molecular weight polyisobutylene having a viscosity average molecular weight of 800,000 to 2,600,000 and low-molecular weight polyisobutylene having a viscosity average molecular weight of 40,000 to 85,000.

7. The preparation of claim 1, wherein a weight ratio of the high-molecular weight polyisobutylene and the low-molecular weight polyisobutylene is 1:0.5 to 1:2.

8. The preparation of claim 1, wherein the adhesive is included in an amount of 35 to 50 parts by weight with respect to a total of 100 parts by weight of the drug-containing matrix layer.

9. The preparation of claim 1, wherein the crystallization inhibitor is polyvinylpyrrolidone.

10. The preparation of claim 1, wherein the crystallization inhibitor is included in an amount of 1.0 to 1.5 parts by weight with respect to a total of 100 parts by weight of the drug-containing matrix layer.

11. The preparation of claim 1, where the donepezil or the pharmaceutically acceptable salt thereof has a skin permeability of 3 to 30 µg/cm²/hr.

12. The preparation of claim 1, wherein the drug-containing matrix layer further comprises one or more additives selected from the group consisting of a stabilizer, a tackifier, and a softening agent.
